# EUROPEAN PATENT APPLICATION

(11) **EP 4 582 520 A1**
(43) Date of publication of application: **09.07.2025**
(21) Application number: 23860437.5
(22) Date of filing: 30.08.2023
(51) Int. Cl.: C10G 2/00, B01D 53/22, C07C 29/152, C07C 31/04, C10L 1/02

(54) **LIQUID FUEL PRODUCTION SYSTEM AND LIQUID FUEL PRODUCTION METHOD**

(30) Priority: 01.09.2022 JP 2022139589
(71) Applicant: NGK Insulators, Ltd., Nagoya-shi, Aichi 467-8530 (JP)
(72) Inventor: IIDA Kazuki, Nagoya-shi, Aichi 467-8530 (JP); MAEHARA Sota, Nagoya-shi, Aichi 467-8530 (JP); ANDO Junichi, Nagoya-shi, Aichi 467-8530 (JP); OKUMA Yusuke, Nagoya-shi, Aichi 467-8530 (JP); KAN Hirofumi, Nagoya-shi, Aichi 467-8530 (JP); SHIBAGAKI Yukinari, Nagoya-shi, Aichi 467-8530 (JP); TAKAHASHI Michio, Nagoya-shi, Aichi 467-8530 (JP)
(74) Representative: Müller-Boré & Partner Patentanwälte PartG mbB
(86) International application number: PCT/JP2023/031603
(87) International publication number: WO 2024/048675

(57) **Abstract**

A primary object of the present invention is to suppress the deterioration of a reaction yield in a conversion reaction from a raw material gas containing hydrogen and a carbon oxide to a liquid fuel. A liquid fuel production system according to an embodiment of the present invention is a liquid fuel production system, including: a liquid fuel-synthesizing portion configured to advance a conversion reaction from a raw material gas containing at least hydrogen and a carbon oxide to a liquid fuel; a raw material gas-supplying portion configured to supply the raw material gas to the liquid fuel-synthesizing portion; and a raw material gas-circulating portion configured to resupply a remaining raw material gas, which contains the hydrogen and the carbon oxide that are unreacted, and an acidic by-product of the conversion reaction, from the liquid fuel-synthesizing portion to the raw material gas-supplying portion, wherein the raw material gas-supplying portion includes a mixing portion configured to mix an amine compound and the remaining raw material gas in the presence of water vapor, and a moisture-removing portion configured to remove a neutralized product of the amine compound and the acidic by-product together with condensed water of the water vapor.

## Description

### Technical Field

The present invention relates to a liquid fuel production system and a method of producing a liquid fuel.

### Background Art

In recent years, the following has been proposed for the purpose of achieving a carbon neutral society: a carbon oxide is grasped as a carbon resource, and is converted into a liquid fuel useful as an industrial basic raw material. In, for example, Patent Literature 1, there is a disclosure of a liquid fuel production system that performs a conversion reaction from a raw material gas containing hydrogen and carbon dioxide to methanol with a membrane reactor including a catalyst and a water vapor separation membrane.

### Citation List

### Patent Literature

[PTL 1] JP 2018-8940 A

### Summary of Invention

### Technical Problem

In such conversion reaction from a raw material gas containing hydrogen and carbon dioxide to a liquid fuel as described above, a reaction yield may deteriorate. Specifically, in the conversion reaction, from the viewpoint of energy savings, the following cyclic use of the raw material gas has been performed: an unreacted raw material gas is recovered from the liquid fuel production system, and is supplied again as the raw material gas to the liquid fuel production system. In the cyclic use of the raw material gas, however, an acidic by-product produced in the conversion reaction may be accumulated along with an increase in number of times of circulation to cause the deterioration of the reaction yield.

A primary object of the present invention is to suppress the deterioration of a reaction yield in a conversion reaction from a raw material gas containing hydrogen and a carbon oxide to a liquid fuel.

### Solution to Problem

[1] According to one aspect of the present invention, there is provided a liquid fuel production system, including: a liquid fuel-synthesizing portion configured to advance a conversion reaction from a raw material gas containing at least hydrogen and a carbon oxide to a liquid fuel; a raw material gas-supplying portion configured to supply the raw material gas to the liquid fuel-synthesizing portion; and a raw material gas-circulating portion configured to resupply a remaining raw material gas, which contains the hydrogen and the carbon oxide that are unreacted, and an acidic by-product of the conversion reaction, from the liquid fuel-synthesizing portion to the raw material gas-supplying portion, wherein the raw material gas-supplying portion includes a mixing portion configured to mix an amine compound and the remaining raw material gas in the presence of water vapor, and a moisture-removing portion configured to remove a neutralized product of the amine compound and the acidic by-product together with condensed water of the water vapor.
[2] In the liquid fuel production system according to the above-mentioned item [1], a neutralizing raw material gas containing the amine compound and the remaining raw material gas may be mixed in the mixing portion.
[3] In the liquid fuel production system according to the above-mentioned item [1], the raw material gas may further contain nitrogen, and the amine compound may be ammonia produced from the hydrogen and the nitrogen in the liquid fuel-synthesizing portion.
[4] The liquid fuel production system according to the above-mentioned item [2] may further include a gas-capturing portion configured to capture carbon dioxide from air or a biogas, and a gas containing the carbon dioxide supplied from the gas-capturing portion may be used as a constituent component for the neutralizing raw material gas.
[5] In The liquid fuel production system according to the above-mentioned item [2] or [4], the liquid fuel-synthesizing portion may include a water vapor separation membrane configured to cause at least water vapor to permeate therethrough, and the liquid fuel-synthesizing portion may separate a permeation-side gas, which is a gas that has permeated through the water vapor separation membrane from a non-permeation side thereof to a permeation side thereof, and which contains water vapor that is a by-product of the conversion reaction, and a non-permeation-side gas, which is a gas that has been free from permeating through the water vapor separation membrane, and which contains the liquid fuel, the hydrogen and the carbon oxide that are unreacted, and the acidic by-product, from each other. In addition, the liquid fuel production system may further include a sweeping gas-supplying portion configured to supply a sweeping gas, which is a gas that sweeps the permeation-side gas, and which contains an amine compound, to the permeation side of the liquid fuel-synthesizing portion, and the permeation-side gas and the sweeping gas that have flowed out of the liquid fuel-synthesizing portion may be supplied to the mixing portion.
[6] In the liquid fuel production system according to the above-mentioned item [5], the sweeping gas may further contain a carbon oxide and/or hydrogen.
[7] In the liquid fuel production system according to the above-mentioned item [5] or [6], a concentration of the amine compound in the sweeping gas may be 10 ppm or more.
[8] In the liquid fuel production system according to the above-mentioned item [3], the liquid fuel-synthesizing portion may include a water vapor separation membrane configured to cause at least water vapor and ammonia to permeate therethrough, and the liquid fuel-synthesizing portion may separate a permeation-side gas, which is a gas that has permeated through the water vapor separation membrane from a non-permeation side thereof to a permeation side thereof, and which contains water vapor that is a by-product of the conversion reaction and the ammonia, and a non-permeation-side gas, which is a gas that has been free from permeating through the water vapor separation membrane, and which contains the liquid fuel, the hydrogen and the carbon oxide that are unreacted, and the acidic by-product, from each other. In addition, the liquid fuel production system may further include a sweeping gas-supplying portion configured to supply a sweeping gas, which sweeps the permeation-side gas, to the permeation side of the liquid fuel-synthesizing portion, and the permeation-side gas and the sweeping gas that have flowed out of the liquid fuel-synthesizing portion may be supplied to the mixing portion.
[9] In the liquid fuel production system according to the above-mentioned item [8], a concentration of the ammonia in a mixed gas of the permeation-side gas and the sweeping gas may be 10 ppm or more.
[10] In the liquid fuel production system according to the above-mentioned item [2] or [4], the liquid fuel-synthesizing portion may include a liquid fuel separation membrane configured to cause at least a liquid fuel to permeate therethrough, and the liquid fuel-synthesizing portion may separate a permeation-side gas, which is a gas that has permeated through the liquid fuel separation membrane from a non-permeation side thereof to a permeation side thereof, and which contains the liquid fuel, and a non-permeation-side gas, which is a gas that has been free from permeating through the liquid fuel separation membrane, and which contains water vapor that is a by-product of the conversion reaction, the hydrogen and the carbon oxide that are unreacted, and the acidic by-product, from each other. In addition, the liquid fuel production system may further include a sweeping gas-supplying portion configured to supply a sweeping gas, which is a gas that sweeps the permeation-side gas, and which contains an amine compound, to the permeation side of the liquid fuel-synthesizing portion, and the sweeping gas that has flowed out of the liquid fuel-synthesizing portion may be supplied to the mixing portion.
[11] In the liquid fuel production system according to the above-mentioned item [10], the sweeping gas may further contain a carbon oxide and/or hydrogen.
[12] In the liquid fuel production system according to the above-mentioned item [10] or [11], a concentration of the amine compound in the sweeping gas may be 10 ppm or more.
[13] In the liquid fuel production system according to the above-mentioned item [3], the liquid fuel-synthesizing portion may include a liquid fuel separation membrane configured to cause at least a liquid fuel and ammonia to permeate therethrough, and the liquid fuel-synthesizing portion may separate a permeation-side gas, which is a gas that has permeated through the liquid fuel separation membrane from a non-permeation side thereof to a permeation side thereof, and which contains the liquid fuel and the ammonia, and a non-permeation-side gas, which is a gas that has been free from permeating through the liquid fuel separation membrane, and which contains water vapor that is a by-product of the conversion reaction, the hydrogen and the carbon oxide that are unreacted, and the acidic by-product, from each other. In addition, the liquid fuel production system may further include a sweeping gas-supplying portion configured to supply a sweeping gas, which sweeps the permeation-side gas, to the permeation side of the liquid fuel-synthesizing portion, and the permeation-side gas and the sweeping gas that have flowed out of the liquid fuel-synthesizing portion may be supplied to the mixing portion.
[14] In the liquid fuel production system according to the above-mentioned item [13], a concentration of the ammonia in a mixed gas of the permeation-side gas and the sweeping gas may be 10 ppm or more.
[15] In the liquid fuel production system according to the above-mentioned item [2], the liquid fuel-synthesizing portion may include a first gas flow path having arranged therein a catalyst that advances the conversion reaction, and a second gas flow path through which a temperature-controlling gas that controls a temperature of the first gas flow path flows. In addition, the liquid fuel production system may further include a temperature-controlling gas-supplying portion configured to supply a temperature-controlling gas containing an amine compound to the second gas flow path of the liquid fuel-synthesizing portion, and the temperature-controlling gas that has flowed out of the liquid fuel-synthesizing portion may be supplied to the mixing portion.
[16] In the liquid fuel production system according to the above-mentioned item [15], the temperature-controlling gas may further contain a carbon oxide and/or hydrogen.
[17] In the liquid fuel production system according to the above-mentioned item [15], a concentration of the amine compound in the temperature-controlling gas may be 10 ppm or more.
[18] According to another aspect of the present invention, there is provided a method of producing a liquid fuel, including: supplying a raw material gas containing at least hydrogen and a carbon oxide to a liquid fuel-synthesizing portion including a catalyst that advances a conversion reaction from the raw material gas to a liquid fuel; advancing the conversion reaction, and recovering a remaining raw material gas, which contains the hydrogen and the carbon oxide that are unreacted, and an acidic by-product, from the liquid fuel-synthesizing portion; removing the acidic by-product from the remaining raw material gas; and resupplying the remaining raw material gas after the removal of the acidic by-product as part of the raw material gas to the liquid fuel-synthesizing portion, wherein the removing the acidic by-product from the remaining raw material gas includes mixing an amine compound and the remaining raw material gas in the presence of water vapor to neutralize the acidic by-product with the amine compound.
[19] In the method of producing a liquid fuel according to the above-mentioned item [18], the removing the acidic by-product from the remaining raw material gas may include mixing a neutralizing raw material gas containing the amine compound after having been supplied to the liquid fuel-synthesizing portion and having flowed through the liquid fuel-synthesizing portion, and the remaining raw material gas.
[20] In the method of producing a liquid fuel according to the above-mentioned item [18], the raw material gas may further contain nitrogen, and the amine compound may be ammonia produced from the hydrogen and the nitrogen in the liquid fuel-synthesizing portion.
[21] In the method of producing a liquid fuel according to the above-mentioned item [19], the neutralizing raw material gas may be prepared by using a gas containing carbon dioxide captured from air or a biogas.
[22] In the method of producing a liquid fuel according to the above-mentioned item [19] or [21], the liquid fuel-synthesizing portion may include a water vapor separation membrane configured to cause at least water vapor to permeate therethrough, water vapor that is a by-product of the conversion reaction may be caused to permeate from a non-permeation side of the water vapor separation membrane to a permeation side thereof, be swept by a sweeping gas containing the amine compound, and be recovered as an exhaust gas from the liquid fuel-synthesizing portion, and the exhaust gas and the remaining raw material gas may be mixed to neutralize the acidic by-product in the remaining raw material gas with the amine compound.
[23] In the method of producing a liquid fuel according to the above-mentioned item [19] or [21], the liquid fuel-synthesizing portion may include a liquid fuel separation membrane configured to cause at least a liquid fuel to permeate therethrough, the liquid fuel may be caused to permeate from a non-permeation side of the liquid fuel separation membrane to a permeation side thereof, be swept by a sweeping gas containing the amine compound, and be recovered as a product gas from the liquid fuel-synthesizing portion, and water vapor that is a by-product of the conversion reaction may be recovered, the product gas may be separated into the liquid fuel and the sweeping gas, and the sweeping gas, the remaining raw material gas, and the water vapor may be mixed to neutralize the acidic by-product in the remaining raw material gas with the amine compound.
[24] In the method of producing a liquid fuel according to the above-mentioned item [19] or [21], a temperature-controlling gas containing the amine compound for controlling a reaction temperature of the conversion reaction may be flowed through the liquid fuel-synthesizing portion, and the temperature-controlling gas after having flowed through the liquid fuel-synthesizing portion and the remaining raw material gas may be mixed to neutralize the acidic by-product in the remaining raw material gas with the amine compound.
[25] In the method of producing a liquid fuel according to the above-mentioned item [20], the liquid fuel-synthesizing portion may include a water vapor separation membrane configured to cause at least water vapor and ammonia to permeate therethrough, water vapor that is a by-product of the conversion reaction and the ammonia may be caused to permeate from a non-permeation side of the water vapor separation membrane to a permeation side thereof, be swept by a sweeping gas, and be recovered as an exhaust gas from the liquid fuel-synthesizing portion, and the exhaust gas and the remaining raw material gas may be mixed to neutralize the acidic by-product in the remaining raw material gas with the ammonia.
[26] In the method of producing a liquid fuel according to the above-mentioned item [20], the liquid fuel-synthesizing portion may include a liquid fuel separation membrane configured to cause at least a liquid fuel and ammonia to permeate therethrough, the liquid fuel and the ammonia may be caused to permeate from a non-permeation side of the liquid fuel separation membrane to a permeation side thereof, be swept by a sweeping gas, and be recovered as a product gas from the liquid fuel-synthesizing portion, and water vapor that is a by-product of the conversion reaction may be recovered together with the remaining raw material gas, and the liquid fuel may be separated from the product gas, and then, the remaining may be mixed with the remaining raw material gas and the water vapor to neutralize the acidic by-product in the remaining raw material gas with the ammonia.

### Advantageous Effects of Invention

According to the liquid fuel production system of the embodiment of the present invention, the deterioration of the reaction yield described above can be suppressed by removing the acidic by-product produced in the conversion reaction through its neutralization in the circulation process of the raw material gas. In addition, corrosion may occur in the liquid fuel production system owing to the acidic by-product. According to the method of producing a liquid fuel of the embodiment of the present invention, however, such corrosion can be prevented.

### Brief Description of Drawings

FIG. **1** is a schematic configuration view of a liquid fuel production system according to one embodiment of the present invention.
FIG. **2** is a schematic configuration view of a liquid fuel production system according to one embodiment of the present invention.
FIG. **3** is a schematic configuration view of a liquid fuel production system according to one embodiment of the present invention.
FIG. **4** is a schematic configuration view of a liquid fuel production system according to one embodiment of the present invention.

### Description of Embodiments

Embodiments of the present invention are described below with reference to the drawings. However, the present invention is not limited to these embodiments. In addition, for clearer illustration, some widths, thicknesses, shapes, and the like of respective portions may be schematically illustrated in the drawings in comparison to the embodiments. However, the widths, the thicknesses, the shapes, and the like are each merely an example, and do not limit the understanding of the present invention.

### A. Liquid Fuel Production System

A liquid fuel production system according to an embodiment of the present invention includes:
a liquid fuel-synthesizing portion configured to advance a conversion reaction from a raw material gas containing at least hydrogen and a carbon oxide to a liquid fuel;
a raw material gas-supplying portion configured to supply the raw material gas to the liquid fuel-synthesizing portion; and
a raw material gas-circulating portion configured to resupply a remaining raw material gas, which contains the hydrogen and the carbon oxide that are unreacted, and an acidic by-product of the conversion reaction, from the liquid fuel-synthesizing portion to the raw material gas-supplying portion.

The raw material gas-supplying portion includes
a mixing portion configured to mix an amine compound and the remaining raw material gas in the presence of water vapor, and
a moisture-removing portion configured to remove a neutralized product of the amine compound and the acidic by-product together with condensed water of the water vapor.

According to the liquid fuel production system of the embodiment of the present invention, the acidic by-product incorporated into the remaining raw material gas is neutralized with the amine compound in the presence of the water vapor, and the produced neutralized product can be removed together with the condensed water of the water vapor (also referred to as "water vapor for removing a neutralized product").

In one embodiment, the liquid fuel-synthesizing portion includes a separation membrane that separates the liquid fuel and water vapor that is a by-product of the conversion reaction (also referred to as "by-product water vapor") from each other.

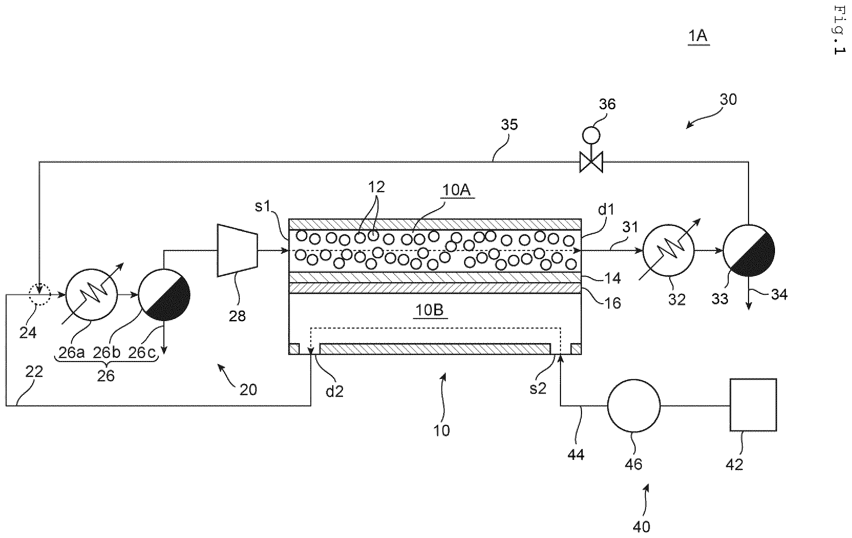

FIG. **1** is a schematic configuration view of the liquid fuel production system according to one embodiment of the present invention.

A liquid fuel production system **1A** illustrated in FIG. **1** includes: a liquid fuel-synthesizing portion **10** configured to advance a conversion reaction from a raw material gas containing at least hydrogen and a carbon oxide to a liquid fuel; a raw material gas-supplying portion **20** configured to supply the raw material gas to the liquid fuel-synthesizing portion **10;** and a raw material gas-circulating portion **30** configured to resupply a remaining raw material gas, which contains hydrogen and the carbon oxide that are unreacted, and the acidic by-product of the conversion reaction, from the liquid fuel-synthesizing portion **10** to the raw material gas-supplying portion **20.**

The liquid fuel-synthesizing portion **10** includes a water vapor separation membrane **14** configured to cause at least water vapor to permeate therethrough, and the liquid fuel-synthesizing portion separates a permeation-side gas, which is a gas that has permeated through the water vapor separation membrane **14** from a non-permeation side thereof to a permeation side thereof, and which contains water vapor that is a by-product of the conversion reaction, and a non-permeation-side gas, which is a gas that has been free from permeating through the water vapor separation membrane **14,** and which contains the liquid fuel, hydrogen and the carbon oxide that are unreacted, and the acidic by-product, from each other.

The liquid fuel production system **1A** further includes a sweeping gas-supplying portion **40** configured to supply a gas that sweeps the permeation-side gas to the liquid fuel-synthesizing portion **10.**

The raw material gas-supplying portion **20** includes: a mixing portion **24** configured to mix an amine compound and the remaining raw material gas in the presence of the water vapor; and a moisture-removing portion **26** configured to remove a neutralized product of the amine compound and the acidic by-product together with the condensed water of the water vapor.

According to the liquid fuel production system **1A,** a gas containing the amine compound (neutralizing raw material gas) is used as the sweeping gas, and hence the sweeping gas (neutralizing raw material gas) flowing out of the liquid fuel-synthesizing portion **10,** the water vapor incorporated into the permeation-side gas, and the remaining raw material gas incorporated into the non-permeation-side gas are mixed. Thus, the acidic by-product in the remaining raw material gas can be neutralized with the amine compound in the presence of the water vapor.

In this description, the liquid fuel is a fuel that is in a liquid state at normal temperature and normal pressure or a fuel that can be liquefied under a normal-temperature pressurized state. Examples of the fuel that is in a liquid state at normal temperature and normal pressure include methanol, ethanol, a liquid fuel represented by CₙH₂₍ₘ₋₂ₙ₎ ("m" represents an integer of less than 90 and "n" represents an integer of less than 30), and a mixture thereof. Examples of the fuel that can be liquefied under a normal-temperature pressurized state include propane, butane, and a mixture thereof.

As an example of the conversion reaction, reactions that may occur at the time of the synthesis of methanol through the catalytic hydrogenation of a raw material gas containing carbon monoxide, carbon dioxide, and hydrogen in the presence of a catalyst are represented by the following reaction formulae.

CO+2H₂ ↔ CH₃OH (1)

CO₂+3H₂ ↔ CH₃OH+H₂O (2)

CO₂+H₂ ↔ CO+H₂O (3)

Each of the reactions is an equilibrium reaction, and hence, to increase both of its conversion ratio and reaction rate, the reaction is preferably performed under high temperature and high pressure. A reaction temperature is, for example, 180°C or more, preferably 200°C or more and 350°C or less, more preferably 200°C or more and 300°C or less. A reaction pressure is, for example, 1 MPa (G) or more, preferably 2.0 MPa (G) or more and 6.0 MPa (G) or less, more preferably 2.5 MPa (G) or more and 4.0 MPa (G) or less. The liquid fuel is in a gas state at the time of its synthesis, and at least until the liquid fuel flows out of the liquid fuel-synthesizing portion, the liquid fuel is maintained while being in a gas state.

### A-1. Liquid Fuel-synthesizing Portion

The liquid fuel-synthesizing portion **10** is a so-called membrane reactor for converting the raw material gas into the liquid fuel. The shape of the liquid fuel-synthesizing portion **10** is not particularly limited, but may be set to, for example, a monolith shape, a flat-plate shape, a tubular shape, a cylindrical shape, a columnar shape, or a polygonal columnar shape. The monolith shape means a shape having a plurality of cells penetrating the shape in its lengthwise direction, and is a concept including a honeycomb shape.

The liquid fuel-synthesizing portion **10** includes: a catalyst **12;** the water vapor separation membrane **14;** a non-permeation-side space **10A;** and a permeation-side space **10B.** In the illustrated example, the water vapor separation membrane **14** is supported by a porous support **16.** In the liquid fuel-synthesizing portion **10,** a first supply port **s1** and a first discharge port **d1** that communicate to each other through the non-permeation-side space **10A,** and a second supply port **s2** and a second discharge port **d2** that communicate to each other through the permeation-side space **10B** are arranged. The liquid fuel-synthesizing portion **10** preferably has heat resistance and pressure resistance suitable for conditions for the synthesis of a desired liquid fuel.

The catalyst **12** advances the conversion reaction from the raw material gas to the liquid fuel. The catalyst is arranged in the non-permeation-side space **10A** on the non-permeation side of the water vapor separation membrane **14.** Although the catalyst is preferably filled into the non-permeation-side space **10A,** the catalyst may be arranged in a layer shape or an island shape on the surface of the water vapor separation membrane **14.** When the catalyst has a particle shape like the illustrated example, the particle diameters (diameters) of catalyst particles may each be set to, for example, 0.5 mm or more and 10 mm or less. Each of the catalyst particles may be formed only of the catalyst, or may have a configuration in which the catalyst is carried by a carrier particle. The carrier particle is preferably a porous particle.

Any catalyst suitable for a conversion reaction to a desired liquid fuel may be used as the catalyst. Specifically, metal catalysts (e.g., copper and palladium), oxide catalysts (e.g., zinc oxide, zirconia, and gallium oxide), and catalysts obtained by compositing these catalysts (e.g., copper-zinc oxide, copper-zinc oxide-alumina, copper-zinc oxide-chromium oxide-alumina, copper-cobalt-titania, and catalysts obtained by modifying these catalysts with palladium) may each be used.

The water vapor separation membrane **14** causes water vapor, which is a by-product of the conversion reaction from the raw material gas to the liquid fuel, to permeate therethrough. Thus, the reaction equilibrium of the formula (2) can be shifted to a product side through utilization of an equilibrium shift effect.

The molecular diameter (0.26 nm) of water is close to the molecular diameter (0.296 nm) of hydrogen. Accordingly, for example, not only the water vapor that is a by-product of the conversion reaction but also part of hydrogen in the raw material gas can permeate through the water vapor separation membrane **14.**

The water vapor separation membrane **14** preferably has a water vapor permeance of 100 nmol/(s·Pa·m²) or more. The water vapor permeance may be determined by an existing method (see Ind. Eng. Chem. Res., 40, 163-175 (2001)).

The water vapor separation membrane **14** preferably has a separation factor of 100 or more. As the separation factor becomes larger, the membrane more easily causes the water vapor to permeate therethrough, and more hardly causes components except the water vapor (e.g., hydrogen, the carbon oxide, oxygen, and the liquid fuel) to permeate therethrough. The separation factor may be determined by an existing method (see Fig. 1 of "Separation and Purification Technology 239 (2020) 116533").

An inorganic membrane may be used as the water vapor separation membrane **14.** The inorganic membrane is preferred because the membrane has heat resistance, pressure resistance, and water vapor resistance. Examples of the inorganic membrane include a zeolite membrane, a silica membrane, an alumina membrane, and a composite membrane thereof. For example, an LTA zeolite membrane in which the molar ratio (Si/Al) of a silicon element (Si) to an aluminum element (Al) is 1.0 or more and 3.0 or less is suitable because the membrane is excellent in water vapor permeability.

The zeolite membrane to be used as the water vapor separation membrane **14** may be obtained by a production method described in, for example, JP 2004-66188 A. In addition, the silica membrane to be used as the water vapor separation membrane **14** may be obtained by a production method described in, for example, WO 2008/050812 A1.

The porous support **16** includes a porous material. For example, a ceramic material, a metal material, a resin material, and a composite member thereof may each be used as the porous material, and the ceramic material is particularly suitable. For example, alumina (Al₂O₃), titania (TiO₂), mullite (Al₂O₃·SiO₂), ceramic fragments, cordierite (Mg₂Al₄Si₅O₁₈), and a composite material containing two or more of these materials may each be used as an aggregate for the ceramic material, and alumina is suitable in consideration of ease of availability, green body stability, and corrosion resistance. At least one of titania, mullite, easily sinterable alumina, silica, glass frit, a clay mineral, or easily sinterable cordierite may be used as an inorganic binder for the ceramic material. The ceramic material may be free of any inorganic binder.

The average pore diameter of the porous support may be set to 5 µm or more and 25 µm or less. The average pore diameter of the porous support may be measured by a mercury intrusion method. The porosity of the porous support may be set to 25% or more and 50% or less. The average particle diameter of the porous material for forming the porous support may be set to 1 µm or more and 100 µm or less. In this embodiment, the average particle diameter is the arithmetic average value of the maximum diameters of 30 measurement object particles (randomly selected) measured by sectional microstructure observation with a scanning electron microscope (SEM).

The non-permeation-side space **10A** is a space on the non-permeation side of the water vapor separation membrane **14.** The raw material gas supplied from the raw material gas-supplying portion **20** flows into the non-permeation-side space **10A** through the first supply port **s1.** In the catalyst **12,** the liquid fuel is synthesized from the raw material gas, and the produced liquid fuel flows out of the non-permeation-side space **10A** to the raw material gas-circulating portion **30** through the first discharge port **d1** together with the raw material gas that is unreacted (remaining raw material gas), as the non-permeation-side gas. The non-permeation-side gas contains the liquid fuel and the remaining raw material gas. The remaining raw material gas further contains the acidic by-product in addition to hydrogen and the carbon oxide that are unreacted. The acidic by-product means a by-product that acts as a so-called Bronsted acid out of the by-products of the conversion reaction. Examples of the acidic by-product include formic acid and methyl formate.

The permeation-side space **10B** is a space on the permeation side of the water vapor separation membrane **14.** The water vapor produced in the conversion reaction, the hydrogen in the raw material gas, and the like permeate through the water vapor separation membrane **14** to flow into the permeation-side space **10B.**

According to the liquid fuel-synthesizing portion **10** having the above-mentioned configuration, the conversion reaction can be advanced by the supply of the raw material gas, and the permeation-side gas, which is a gas that has permeated through the water vapor separation membrane from the non-permeation-side space **10A** to the permeation-side space **10B,** and which contains the water vapor that is a by-product of the conversion reaction, and the non-permeation-side gas, which is a gas that has not permeated therethrough, and which contains the liquid fuel, hydrogen and the carbon oxide that are unreacted, and the acidic by-product, can be separated from each other.

In addition, the sweeping gas is supplied from the sweeping gas-supplying portion **40** to the permeation-side space **10B** through the second supply port **s2.** The permeation-side gas and the sweeping gas flow as an exhaust gas out of the permeation-side space **10B** to the raw material gas-supplying portion **20** through the second discharge port **d2.**

### A-2. Sweeping Gas-supplying Portion

The sweeping gas-supplying portion **40** is arranged on the upstream side of the permeation-side space **10B.** The sweeping gas-supplying portion **40** includes: a sweeping gas-storing portion **42;** a sweeping gas-supplying pipe **44** that connects the sweeping gas-storing portion **42** and the second inflow port **s2** of the liquid fuel-synthesizing portion **10** to each other; and a heating portion **46** interposed in the sweeping gas-supplying pipe **44.**

The sweeping gas-storing portion **42** stores the sweeping gas. The sweeping gas is heated to a desired temperature (e.g., 150°C or more and 350°C or less) in the heating portion **46,** and is then supplied from the second inflow port **s2** to the liquid fuel-synthesizing portion **10.** The heating portion **46** only needs to be capable of heating the sweeping gas, and is hence not particularly limited.

The sweeping gas contains the amine compound, and preferably further contains one or both of hydrogen and the carbon oxide. As described later for the raw material gas-supplying portion, in this embodiment, the raw material gas is prepared by using a mixed gas of the exhaust gas containing the sweeping gas and the remaining raw material gas. Herein, the sweeping gas contains the amine compound, and hence the acidic by-product in the remaining raw material gas can be removed by being neutralized with the amine compound.

Any appropriate amine compound may be used as the amine compound as long as the effects of the present invention are obtained. An amino group of the amine compound except ammonia may be a primary amino group, a secondary amino group, a tertiary amino group, or a combination of two or more thereof.

In one embodiment, an amine compound having a boiling point of 100°C or more and 500°C or less, preferably 170°C or more and 350°C or less is used. Such amine compound can satisfactorily maintain its gas state in the sweeping gas.

Specific examples of the amine compound include ammonia, polyethylenimine, monoethanolamine, diethanolamine, triethanolamine, tetraethyleneaminepentamine, methyldiethanolamine, dibutylamine, ethylenediamine, diethylenetriamine, triethylenetetramine, hexaethylenediamine, benzylamine, N-(3-aminopropyl)diethanolamine, aminopropyltrimethoxysilane, polyvinylamine, 3-aminopropyltriethoxysilane, N-(2-aminoethyl)-3-aminopropyltrimethoxysilane, and other aminosilane coupling agents. The amine compounds may be used alone or in combination thereof.

The concentration of the amine compound in the sweeping gas is not limited as long as the effects of the present invention are obtained. From the viewpoint of neutralizing the acidic by-product, the concentration of the amine compound in the sweeping gas is, for example, 10 ppm or more. In addition, the upper limit of the concentration of the amine compound is not particularly limited. However, some catalysts are inhibited by an alkali, and hence the concentration may be appropriately adjusted to such a concentration that a reduction in catalytic action is prevented. The concentration of the amine compound in the sweeping gas is the concentration of the amine compound in the sweeping gas at the second supply port **s2** of the liquid fuel-synthesizing portion.

In one embodiment, the sweeping gas contains hydrogen or the carbon oxide as a main component, and preferably contains hydrogen as a main component. The phrase "contains hydrogen or the carbon oxide as a main component" means that the concentration of hydrogen or the carbon oxide in the sweeping gas is highest. The concentration of hydrogen in the sweeping gas is, for example, about 50 vol% or more and about 90 vol% or less, preferably 65 vol% or more and 80 vol% or less. The concentration of the carbon oxide is, for example, about 10 vol% or more and less than about 50 vol%, preferably 20 vol% or more and 35 vol% or less. When the sweeping gas is formed substantially only of hydrogen, the carbon oxide, and the amine compound, the exhaust gas may be suitably reused as part of the raw material gas without being separated into hydrogen derived from the raw material gas and the sweeping gas.

### A-3. Raw Material Gas-circulating Portion

The raw material gas-circulating portion **30** includes: a non-permeation-side gas-recovering pipe **31;** a first condenser **32;** a first drain trap **33;** a liquid fuel-recovering pipe **34;** and a raw material gas-circulating pipe **35.** One end of the non-permeation-side gas-recovering pipe **31** is connected to the first outflow port **d1** of the liquid fuel-synthesizing portion **10,** and the other end thereof is connected to the first condenser **32.** One end of the raw material gas-circulating pipe **35** is connected to the first drain trap **33,** and the other end thereof is connected to the raw material gas-supplying portion 20 (more specifically, the mixing portion **24**).

The non-permeation-side gas recovered from the liquid fuel-synthesizing portion **10** through the non-permeation-side gas-recovering pipe **31** is supplied to the first condenser **32.** Thus, the liquid fuel is condensed (liquefied). The liquefied liquid fuel is separated from the remaining raw material gas by the first drain trap **33,** and is recovered from the liquid fuel-recovering pipe **34.**

The non-permeation-side gas after the separation of the liquid fuel (in other words, the remaining raw material gas) contains a trace amount of the acidic by-product in addition to hydrogen and the carbon oxide that are unreacted. The remaining raw material gas is supplied to the raw material gas-supplying portion **20** (more specifically, the mixing portion **24**) through the raw material gas-circulating pipe **35.**

A purge valve **36** may be arranged in the raw material gas-circulating pipe **35** like the illustrated example. The purge valves **36** are arranged at any one or more appropriate positions of the raw material gas-circulating pipe **35** to discharge part of the remaining raw material gas flowing through the raw material gas-circulating pipe **35** to the outside.

### A-4. Raw Material Gas-supplying Portion

The raw material gas-supplying portion **20** includes: a neutralizing raw material gas-supplying pipe **22;** the mixing portion **24;** the moisture-removing portion **26;** and a pressure-boosting portion **28.** The moisture-removing portion **26** includes: a second condenser **26a;** a second drain trap **26b;** and a moisture-capturing pipe **26c.** One end of the neutralizing raw material gas-supplying pipe **22** is connected to the second outflow port **d2** of the liquid fuel-synthesizing portion **10,** and the other end thereof is connected to the second condenser **26a** of the moisture-removing portion **26.** In addition, the raw material gas-circulating pipe **35** is connected to the neutralizing raw material gas-supplying pipe **22** so as to merge therewith on the upstream of the moisture-removing portion **26,** and the connection site serves as the mixing portion **24.** The mixing portion only needs to have such a configuration as to be capable of mixing the neutralizing raw material gas and the remaining raw material gas. For example, the following may be performed: a tank (mixing portion) is arranged on the upstream of the moisture-removing portion **26;** the neutralizing raw material gas-supplying pipe **22** and the raw material gas-circulating pipe **35** are each independently connected to the tank; and the neutralizing raw material gas and the remaining raw material gas are mixed in the tank.

According to the above-mentioned configuration, the exhaust gas (the permeation-side gas and the sweeping gas) recovered from the liquid fuel-synthesizing portion **10** through the neutralizing raw material gas-supplying pipe **22** is mixed with the remaining raw material gas on the upstream of the moisture-removing portion **26.** Thus, the acidic by-product incorporated into the remaining raw material gas is neutralized with the amine compound (amine compound derived from the sweeping gas) incorporated into the exhaust gas. In addition, when the mixed gas of the exhaust gas and the remaining raw material gas is supplied to the second condenser **26a,** the water vapor (water vapor derived from the permeation-side gas) incorporated into the exhaust gas is condensed, and a neutralized product produced by the neutralization migrates to (is dissolved in) the condensed water. Water is produced along with the neutralization to increase the amount of the condensed water, and as a result, the dissolution amount of the neutralized product increases. Accordingly, the neutralized product can efficiently migrate to the condensed water. The condensed water containing the neutralized product is separated from the remaining gas component by the second drain trap **26b,** and is recovered (removed) from the moisture-capturing pipe **26c.** The concentration of the amine compound in the exhaust gas (the concentration of the amine compound at the second discharge port **d2** of the liquid fuel-synthesizing portion) is, for example, 10 ppm or more. The upper limit value of the concentration of the amine compound in the exhaust gas is not particularly limited. However, some catalysts are inhibited by an alkali, and hence the concentration may be appropriately adjusted to such a concentration that a reduction in catalytic action is prevented.

The gas component after the separation of the condensed water and the neutralized product is increased in pressure and temperature in the pressure-boosting portion **28,** and is then supplied as the raw material gas to the non-permeation-side space **10A** of the liquid fuel-synthesizing portion **10.** The raw material gas is obtained through the cyclic use of the remaining raw material gas, but is suppressed in accumulation of the acidic by-product along with an increase in number of times of circulation. In addition, the gas component after the separation of the condensed water and the neutralized product may be mixed with hydrogen and/or the carbon oxide as required so that the raw material gas may be prepared in desired composition, though a process therefor is not shown.

As described above, the raw material gas contains at least hydrogen and the carbon oxide. The concentration of the carbon oxide in the raw material gas is, for example, 10 vol% or more and 40 vol% or less, preferably 20 vol% or more and 30 vol% or less. The concentration of hydrogen in the raw material gas is, for example, 60 vol% or more and 90 vol% or less, preferably 70 vol% or more and 80 vol% or less.

### A-5. Modification Example 1

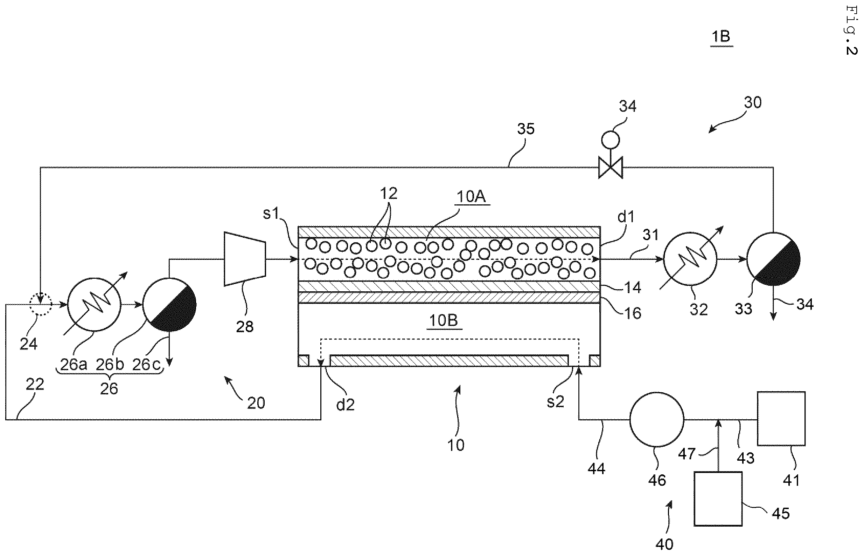

FIG. **2** is a schematic configuration view of a liquid fuel production system according to another embodiment of the present invention.

A liquid fuel production system **1B** illustrated in FIG. **2** includes: the liquid fuel-synthesizing portion **10;** the raw material gas-supplying portion **20;** the raw material gas-circulating portion **30;** and the sweeping gas-supplying portion **40.** The liquid fuel production system **1B** is different from the liquid fuel production system **1A** illustrated in FIG. **1** in that the sweeping gas-supplying portion **40** includes: a carbon dioxide-capturing portion **41;** a carbon dioxide-supplying pipe **43;** a hydrogen-producing portion **45;** a hydrogen-supplying pipe **47;** the sweeping gas-supplying pipe **44;** and the heating portion **46.**

For example, the carbon dioxide-capturing portion **41** includes a carbon dioxide adsorbent, which adsorbs carbon dioxide by being brought into contact with a carbon dioxide-containing gas, and which desorbs carbon dioxide through heating, a pressure reduction, or the like, and the portion can capture a gas (carbon dioxide-enriched gas) containing carbon dioxide at a concentration higher than that of the carbon dioxide-containing gas with the carbon dioxide adsorbent. More specifically, the carbon dioxide-capturing portion **41** can capture the carbon dioxide-enriched gas as follows: the portion brings the carbon dioxide-containing gas into contact with the carbon dioxide adsorbent to cause the adsorbent to adsorb carbon dioxide; and then, the portion heats the carbon dioxide adsorbent and/or reduces the pressure therein, to cause the adsorbent to desorb carbon dioxide, and sucks the desorbed carbon dioxide with a pump or the like. In one embodiment, the carbon dioxide-capturing portion **41** is a carbon dioxide-capturing facility utilizing a direct air capture (DAC) technology. An amine compound is typically used as the carbon dioxide adsorbent. Specific examples of the amine compound are as described above for the sweeping gas. The carbon dioxide-enriched gas may contain a trace amount of the carbon dioxide adsorbent, that is, the amine compound owing to the inclusion of the carbon dioxide adsorbent at the time of the capture of the carbon dioxide-enriched gas. In addition, the carbon dioxide-enriched gas may contain nitrogen derived from air.

In addition, the carbon dioxide-capturing portion **41** may be, for example, a carbon dioxide-capturing facility that captures carbon dioxide from a biogas with a separation membrane. The biogas is a gas produced through fermentation (methane fermentation) by using biomass, such as garbage, waste paper, or livestock manure, as a raw material. The main components of the biogas are methane and carbon dioxide, and the biogas may further contain, for example, a trace amount of nitrogen.

The hydrogen-producing portion **45** is, for example, a hydrogen-producing facility utilizing a water electrolysis technology.

The carbon dioxide-supplying pipe **43** connects the carbon dioxide-capturing portion **41** and the heating portion **46** to each other, and the hydrogen-supplying pipe **47** connects the hydrogen-producing portion **45** and the carbon dioxide-supplying pipe **43** to each other. Thus, the carbon dioxide-enriched gas supplied from the carbon dioxide-capturing portion **41** and hydrogen supplied from the hydrogen-producing portion **45** are mixed to produce the sweeping gas. The sweeping gas is heated in the heating portion **46,** and is then supplied to the liquid fuel-synthesizing portion **10** through the sweeping gas-supplying pipe **44.** That is, the carbon dioxide-enriched gas is supplied as a constituent component for the sweeping gas (neutralizing raw material gas) to the raw material gas-supplying portion **10.** However, the total amount of carbon dioxide incorporated into the sweeping gas may be derived from the carbon dioxide-enriched gas (e.g., a carbon dioxide-enriched gas captured from air by the DAC or a carbon dioxide-enriched gas captured from a biogas with a separation membrane), or only part thereof may be derived from the carbon dioxide-enriched gas. As described above, the carbon dioxide-enriched gas may contain a trace amount of the amine compound. Accordingly, a mixed gas of the carbon dioxide-enriched gas and hydrogen may be used as the sweeping gas without separate addition of the amine compound or merely by adding an extremely small amount of the amine compound.

### A-6. Modification Example 2

A separation membrane that separates water vapor and the liquid fuel from each other is not limited to a water vapor separation membrane that causes the water vapor to permeate therethrough, and a liquid fuel separation membrane configured to cause at least the liquid fuel to permeate therethrough may be used. In the present invention, the separation membrane that separates the water vapor and the liquid fuel from each other has higher selective permeability for one of the water vapor and the liquid fuel than for the other thereof, and may not completely separate both the water vapor and the liquid fuel from each other as long as the effects of the present invention are obtained. For example, the liquid fuel separation membrane causes the liquid fuel to permeate therethrough with selectivity higher than that of the water vapor, and does not completely separate both the water vapor and the liquid fuel from each other.

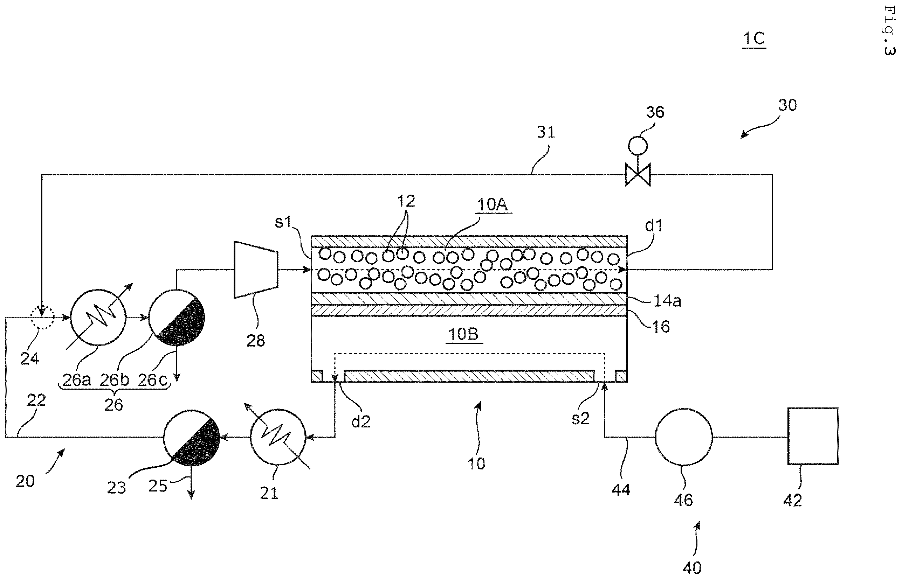

FIG. **3** is a schematic configuration view of a liquid fuel production system according to another embodiment of the present invention.

A liquid fuel production system **1C** illustrated in FIG. **3** includes: the liquid fuel-synthesizing portion **10** configured to advance a conversion reaction from a raw material gas containing at least hydrogen and a carbon oxide to a liquid fuel; the raw material gas-supplying portion **20** configured to supply the raw material gas to the liquid fuel-synthesizing portion **10;** and the raw material gas-circulating portion **30** configured to resupply a remaining raw material gas, which contains hydrogen and the carbon oxide that are unreacted, and the acidic by-product of the conversion reaction, from the liquid fuel-synthesizing portion **10** to the raw material gas-supplying portion **20.**

The liquid fuel-synthesizing portion **10** includes a liquid fuel separation membrane **14a** that causes the liquid fuel to permeate therethrough, and the portion separates a permeation-side gas, which is a gas that has permeated through the liquid fuel separation membrane **14a** from its non-permeation side to its permeation side, and which contains at least the liquid fuel, and a non-permeation-side gas, which is a gas that has not permeated through the liquid fuel separation membrane **14a,** and which contains by-product water vapor, hydrogen and the carbon oxide that are unreacted, and the acidic by-product, from each other.

The liquid fuel production system **1C** further includes the sweeping gas-supplying portion **40** configured to supply a gas that sweeps the permeation-side gas to the permeation side of the liquid fuel-synthesizing portion **10.**

The raw material gas-supplying portion **20** includes: the mixing portion **24** configured to mix an amine compound and the remaining raw material gas in the presence of the water vapor; and the moisture-removing portion **26** configured to remove a neutralized product of the amine compound and the acidic by-product together with the condensed water of the water vapor.

According to the liquid fuel production system **1C,** a gas containing the amine compound (neutralizing raw material gas) is used as a sweeping gas, and hence the sweeping gas (neutralizing raw material gas) flowing out of the liquid fuel-synthesizing portion **10,** and the non-permeation-side gas containing the by-product water vapor and the remaining raw material gas are mixed. Thus, the acidic by-product in the remaining raw material gas can be neutralized with the amine compound. The main point of Modification Example 2 is described below. The other points thereof are as described for the liquid fuel production system **1A.**

The liquid fuel-synthesizing portion **10** includes: the catalyst **12;** the liquid fuel separation membrane **14a;** the non-permeation-side space **10A;** and the permeation-side space **10B.** The liquid fuel separation membrane **14a** may be supported by the porous support **16** like the illustrated example. In the liquid fuel-synthesizing portion **10,** the first supply port **s1** and the first discharge port **d1** that communicate to each other through the non-permeation-side space **10A,** and the second supply port **s2** and the second discharge port **d2** that communicate to each other through the permeation-side space **10B** are arranged. A membrane described in, for example, JP 2020-23488 A may be used as a separation membrane that selectively causes the liquid fuel to permeate therethrough.

The non-permeation-side space **10A** is a space on the non-permeation side of the liquid fuel separation membrane **14a,** and the permeation-side space **10B** is a space on the permeation side of the liquid fuel separation membrane **14a.** The raw material gas supplied from the raw material gas-supplying portion **20** flows into the non-permeation-side space **10A** through the first supply port **s1.** In the catalyst **12,** the liquid fuel is synthesized from the raw material gas, and the liquid fuel permeates through the liquid fuel separation membrane **14a** to flow into the permeation-side space **10B.** The sweeping gas is supplied from the sweeping gas-supplying portion **40** to the permeation-side space **10B** through the second supply port **s2.** The permeation-side gas and the sweeping gas flow as a product gas out of the permeation-side space **10B** to the raw material gas-supplying portion **20** through the second discharge port **d2.** Meanwhile, the remaining raw material gas flows as the non-permeation-side gas out of the non-permeation-side space **10A** to the raw material gas-circulating portion **30** through the first discharge port **d1** together with the by-product water vapor.

The raw material gas-circulating portion **30** includes the non-permeation-side gas-recovering pipe **31.** One end of the non-permeation-side gas-recovering pipe **31** is connected to the first outflow port **d1** of the liquid fuel-synthesizing portion **10,** and the other end thereof is connected to the neutralizing raw material gas-supplying pipe **22.** Thus, the non-permeation-side gas can be supplied to the mixing portion **24.**

The raw material gas-supplying portion **20** includes: the neutralizing raw material gas-supplying pipe **22;** the mixing portion **24;** the moisture-removing portion **26;** the pressure-boosting portion **28;** a third condenser **21;** a third drain trap **23;** and a liquid fuel-recovering pipe **25.** The product gas recovered from the liquid fuel-synthesizing portion **10** through the neutralizing raw material gas-supplying pipe **22** is subjected to gas-liquid separation into the liquid fuel and the sweeping gas by the third condenser **21** and the third drain trap **23,** and the liquefied liquid fuel is recovered through the liquid fuel-recovering pipe **25.** The sweeping gas is delivered to the moisture-removing portion **26,** and is mixed with the non-permeation-side gas (in other words, the remaining raw material gas and the by-product water vapor) in the mixing portion **24** on the upstream of the moisture-removing portion **26.** Thus, the acidic by-product incorporated into the remaining raw material gas is neutralized with the amine compound incorporated into the sweeping gas. In addition, when the mixed gas of the sweeping gas and the non-permeation-side gas is supplied to the second condenser **26a,** the by-product water vapor derived from the non-permeation-side gas is condensed, and the neutralized product produced by the neutralization migrates to (is dissolved in) the condensed water. The condensed water containing the neutralized product is separated from the remaining gas component by the second drain trap **26b,** and is recovered (removed) from the moisture-capturing pipe **26c.**

According to the liquid fuel separation membrane that does not completely separate the liquid fuel and water vapor from each other, the permeation-side gas may contain the liquid fuel and the by-product water vapor, and hence the product gas may contain the sweeping gas containing the amine compound, and the liquid fuel and the water vapor. Accordingly, even when the non-permeation-side gas is free of water vapor, or when the water vapor is separated from the non-permeation-side gas, the mixing of the amine compound and the remaining raw material gas in the presence of the water vapor may be performed by mixing the product gas after the separation of the liquid fuel and the remaining raw material gas. As described above, in the embodiment using the liquid fuel separation membrane, the water vapor present at the time of the mixing of the amine compound and the remaining raw material gas may be water vapor which is recovered together with the remaining raw material gas and incorporated into the non-permeation-side gas and/or water vapor which is recovered as the permeation-side gas and incorporated into the product gas.

### A-7. Modification Example 3

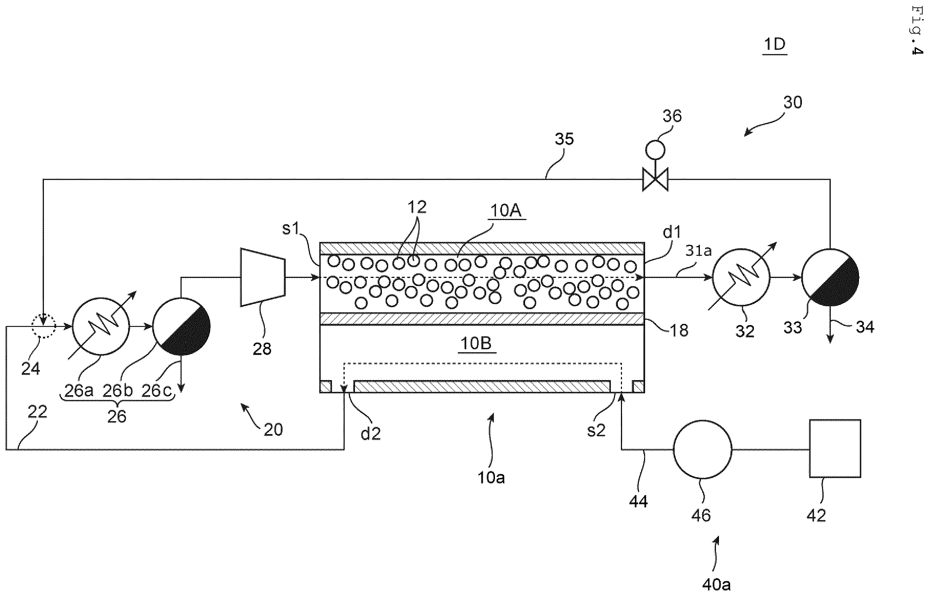

FIG. **4** is a schematic configuration view of a liquid fuel production system according to another embodiment of the present invention.

A liquid fuel production system **1D** illustrated in FIG. **4** includes: a liquid fuel-synthesizing portion **10a** configured to advance a conversion reaction from a raw material gas containing at least hydrogen and a carbon oxide to a liquid fuel; the raw material gas-supplying portion **20** configured to supply the raw material gas to the liquid fuel-synthesizing portion **10a;** and the raw material gas-circulating portion **30** configured to recover a remaining raw material gas, which contains hydrogen and the carbon oxide that are unreacted, and the acidic by-product of the conversion reaction, from the liquid fuel-synthesizing portion **10a,** and to supply the gas to the raw material gas-supplying portion **20.**

The liquid fuel production system **1D** further includes a temperature-controlling gas-supplying portion **40a** that supplies a temperature-controlling gas, which controls the temperature of a first gas flow path **10A,** to the liquid fuel-synthesizing portion **10a.**

The raw material gas-supplying portion **20** includes: the mixing portion **24** configured to mix an amine compound and the remaining raw material gas in the presence of the water vapor; and the moisture-removing portion **26** configured to remove a neutralized product of the amine compound and the acidic by-product together with the condensed water of the water vapor.

According to the liquid fuel production system **1D,** a gas containing the water vapor and the amine compound (neutralizing raw material gas) is used as the temperature-controlling gas, and hence the temperature-controlling gas flowing out of the liquid fuel-synthesizing portion **10a** is mixed with the remaining raw material gas. Thus, the acidic by-product in the remaining raw material gas can be neutralized with the amine compound and removed together with the condensed water.

The liquid fuel-synthesizing portion **10a** includes: the first gas flow path **10A** having arranged therein the catalyst **12** that advances the conversion reaction; and a second gas flow path **10B** through which the temperature-controlling gas that controls the temperature of the first gas flow path **10A** flows. In the illustrated example, the first gas flow path **10A** and the second gas flow path **10B** are vertically divided through a partition **18.** However, the second gas flow path **10B** only needs to be arranged so as to be capable of controlling the temperature of the first gas flow path **10A.** For example, the liquid fuel-synthesizing portion may have such a double tube structure that one of an inner tube or an outer tube is the first gas flow path, and the other thereof is the second gas flow path. The partition **18** is typically gas-impermeable.

The raw material gas supplied from the raw material gas-supplying portion **20** flows into the first gas flow path **10A** through the first supply port **s1.** In the catalyst **12,** the liquid fuel is synthesized from the raw material gas, and the produced liquid fuel flows as a product gas out of the first gas flow path **10A** to the raw material gas-circulating portion **30** through the first discharge port **d1** together with the remaining raw material gas. The product gas contains the liquid fuel, water vapor that is a by-product of the conversion reaction, and the remaining raw material gas. The remaining raw material gas further contains the acidic by-product in addition to hydrogen and the carbon oxide that are unreacted.

The temperature-controlling gas supplied from the temperature-controlling gas-supplying portion **40a** flows into the second gas flow path **10B** through the second supply port **s2,** and flows out to the raw material gas-supplying portion **20** through the second discharge port **d2.**

The temperature-controlling gas-supplying portion **40a** includes: a temperature-controlling gas-storing portion **42** storing the temperature-controlling gas; a temperature-controlling gas-supplying pipe **44** that connects the temperature-controlling gas-storing portion **42** and the second inflow port **s2** of the liquid fuel-synthesizing portion **10a** to each other; and the heating portion **46** interposed in the temperature-controlling gas-supplying pipe **44.** In the illustrated example, the temperature-controlling gas is supplied to the liquid fuel-synthesizing portion **10a** so as to be a counterflow with respect to the raw material gas. However, the temperature-controlling gas may be supplied so as to be a parallel flow with respect thereto.

The temperature-controlling gas is heated to a desired temperature (e.g., 150°C or more and 350°C or less) in the heating portion **46,** and is then supplied from the second inflow port **s2** to the liquid fuel-synthesizing portion **10a.** The heating portion **46** only needs to be capable of heating the temperature-controlling gas, and is hence not particularly limited.

The temperature-controlling gas contains the amine compound and the water vapor, and preferably further contains one or both of hydrogen and the carbon oxide. The same description as that of the sweeping gas may be applied to a component of the temperature-controlling gas except the water vapor. The dew point of the water vapor in the temperature-controlling gas is, for example, 40°C or more and 150°C or less.

The same descriptions as those of the section A-4 and the section A-5 may be applied to the raw material gas-supplying portion **20** and the raw material gas-circulating portion **30.** Specifically, the raw material gas-circulating portion **30** includes: a product gas-recovering pipe **31a;** the first condenser **32;** the first drain trap **33;** the liquid fuel-recovering pipe **34;** and the raw material gas-circulating pipe **35.** The raw material gas-supplying portion **20** includes: the neutralizing raw material gas-supplying pipe **22;** the mixing portion **24;** the moisture-removing portion **26;** and the pressure-boosting portion **28.**

The product gas recovered through the product gas-recovering pipe **31a** contains: the liquid fuel; the by-product water vapor; and the remaining raw material gas containing hydrogen and the carbon oxide that are unreacted, and the acidic by-product. The product gas is subjected to gas-liquid separation by the first condenser **32** and the first drain trap **33,** and the liquefied liquid fuel and water are recovered through the liquid fuel-recovering pipe **34.** In addition, the remaining raw material gas is supplied to the raw material gas-supplying portion **20** (more specifically, the mixing portion **24**) through the raw material gas-circulating pipe **35.** The liquid fuel and the water recovered from the liquid fuel-recovering pipe **34** are further separated and recovered as required.

Meanwhile, in the raw material gas-supplying portion **20,** the temperature-controlling gas containing the amine compound and the water vapor, which has been recovered from the liquid fuel-synthesizing portion **10a** through the neutralizing raw material gas-supplying pipe **22,** is mixed with the remaining raw material gas on the upstream of the moisture-removing portion **26.** Thus, the acidic by-product incorporated into the remaining raw material gas is neutralized with the amine compound incorporated into the temperature-controlling gas, and the neutralized product produced by the neutralization is removed from the moisture-removing portion **26** together with the condensed water of the water vapor. The total amount of the water vapor produced by the conversion reaction may not be recovered in the liquid fuel-recovering pipe **34** owing to a difference in boiling point between the water vapor and methanol, and hence part of the by-product water vapor may be supplied to the mixing portion **24** through the raw material gas-circulating pipe **35.** Accordingly, even when the temperature-controlling gas is free of water vapor, the neutralization of the amine compound and the acidic by-product may be performed in the presence of the by-product water vapor supplied through the raw material gas-circulating pipe **35.**

The gas component after the separation of the condensed water and the neutralized product is increased in pressure and temperature in the pressure-boosting portion **28,** and is then supplied as the raw material gas to the first gas flow path **10A** of the liquid fuel-synthesizing portion **10a.**

### A-8. Modification Example 4

The amine compound to be used in the neutralization of the acidic by-product may be ammonia to be produced from hydrogen and nitrogen in the liquid fuel-synthesizing portion when a raw material gas containing the nitrogen in addition to the hydrogen and the carbon oxide is used. Ammonia has a small molecular diameter, and hence easily permeates through a separation membrane as compared to any other gas. Accordingly, for example, when the liquid fuel-synthesizing portion includes a water vapor separation membrane, ammonia produced in the non-permeation-side space of the liquid fuel-synthesizing portion permeates as a permeation-side gas through the water vapor separation membrane together with water vapor, and may be swept by the sweeping gas to flow as an exhaust gas out of the liquid fuel-synthesizing portion (more specifically, through the discharge port **d2** of the permeation-side space). Accordingly, the exhaust gas (mixed gas of the permeation-side gas and the sweeping gas) contains the water vapor and ammonia. When such exhaust gas is supplied to the mixing portion and mixed with the remaining raw material gas, the acidic by-product in the remaining raw material gas can be neutralized with ammonia in the permeation-side gas in the presence of the water vapor. In addition, for example, when the liquid fuel-synthesizing portion includes a liquid fuel separation membrane, ammonia produced in the non-permeation-side space of the liquid fuel-synthesizing portion permeates as a permeation-side gas through the liquid fuel separation membrane together with the liquid fuel, and may be swept by the sweeping gas to flow as a product gas out of the liquid fuel-synthesizing portion (more specifically, through the discharge port **d2** of the permeation-side space). Accordingly, the product gas (mixed gas of the permeation-side gas and the sweeping gas) contains the liquid fuel and ammonia. In addition, the non-permeation-side gas that has flowed out of the liquid fuel-synthesizing portion through the discharge port **d1** of its non-permeation-side space contains the water vapor and the remaining raw material gas. Accordingly, when the liquid fuel is separated from the product gas, and then the remaining is supplied to the mixing portion and mixed with the non-permeation-side gas, the acidic by-product in the remaining raw material gas can be neutralized with ammonia in the permeation-side gas in the presence of the water vapor. In addition, according to a liquid fuel separation membrane that does not completely separate the liquid fuel and the water vapor from each other, ammonia produced in the non-permeation-side space of the liquid fuel-synthesizing portion permeates as a permeation-side gas through the liquid fuel separation membrane together with the liquid fuel and the water vapor, and may be swept by the sweeping gas to flow as a product gas out of the liquid fuel-synthesizing portion. Accordingly, even when the non-permeation-side gas is free of water vapor, or when the water vapor is separated from the non-permeation-side gas, the acidic by-product in the remaining raw material gas can be neutralized with ammonia in the permeation-side gas in the presence of the water vapor by: supplying the product gas after the separation of the liquid fuel and the remaining raw material gas to the mixing portion; and mixing the gases.

The concentration of ammonia in the exhaust gas or the product gas is, for example, 10 ppm or more, and is, for example, less than 10,000 ppm. The concentration of ammonia is a concentration at the discharge port **d2** of the liquid fuel-synthesizing portion, and is a concentration calculated by excluding a condensable component in the exhaust gas or the product gas.

In addition, the liquid fuel production system of Modification Example 4 may be configured as follows: the system includes such carbon dioxide-capturing portion as described in Modification Example 1, and prepares the raw material gas by using a gas containing carbon dioxide captured from air or a biogas by the carbon dioxide-capturing portion. The gas containing carbon dioxide captured from the air or the biogas may contain a small amount of nitrogen in addition to carbon dioxide. Accordingly, the raw material gas can be prepared without separate addition of nitrogen or merely by adding a small amount of nitrogen.

### B. Method of producing Liquid Fuel

A method of producing a liquid fuel according to an embodiment of the present invention includes:
supplying a raw material gas containing at least hydrogen and a carbon oxide to a liquid fuel-synthesizing portion including a catalyst that advances a conversion reaction from the raw material gas to a liquid fuel (step I);
advancing the conversion reaction, and recovering a remaining raw material gas, which contains the hydrogen and the carbon oxide that are unreacted, and an acidic by-product, from the liquid fuel-synthesizing portion (step II);
removing the acidic by-product from the remaining raw material gas (step III); and
resupplying the remaining raw material gas after the removal of the acidic by-product as part of the raw material gas to the liquid fuel-synthesizing portion (step IV).

Typically, the removing the acidic by-product from the remaining raw material gas is performed by mixing an amine compound and the remaining raw material gas in the presence of water vapor to neutralize the acidic by-product in the remaining raw material gas with the amine compound. A neutralized product produced by the neutralization is removed together with the condensed water of the water vapor. A gas after the removal of the condensed water and the neutralized product from a mixed gas of those components is preferably supplied as a constituent component for the raw material gas to the liquid fuel-synthesizing portion.

In a method of producing a liquid fuel in which an unreacted raw material gas is recovered and circulated to be utilized as the raw material gas again, the concentration of the acidic by-product in the raw material gas may gradually increase along with an increase in number of times of circulation to cause a reduction in yield of the liquid fuel or the corrosion of a liquid fuel production system. In contrast, according to the method of producing a liquid fuel of the embodiment of the present invention, the acidic by-product can be neutralized and removed with the amine compound by mixing the amine compound with the remaining raw material gas, and a problem of the concentration of the acidic by-product can be prevented by utilizing the mixed gas after the removal as a constituent component for the raw material gas.

The method of producing a liquid fuel according to the embodiment of the present invention may be suitably performed with the liquid fuel production system described in the section A. An embodiment in which the liquid fuel production system **1A,** whose liquid fuel-synthesizing portion includes the water vapor separation membrane, and which uses the sweeping gas containing the amine compound as the neutralizing raw material gas, is used is described below. Needless to say, however, the method of producing a liquid fuel according to the embodiment of the present invention may be performed in accordance with the description of the section A even when, for example, a liquid fuel production system whose liquid fuel-synthesizing portion includes a liquid fuel separation membrane, or a liquid fuel production system, which includes a separation membrane, and uses a raw material gas containing nitrogen in addition to the carbon oxide and hydrogen, is used. In addition, even in the case where the liquid fuel production system **1D** is used, and the temperature-controlling gas after having been flowed through the liquid fuel-synthesizing portion for controlling the reaction temperature of the conversion reaction is used as the neutralizing raw material gas, the method of producing a liquid fuel according to the embodiment of the present invention may be performed in the same manner as that in the case where the liquid fuel production system **1A** is used. For example, the following may be performed: a temperature-controlling gas containing the amine compound and water vapor is flowed through the liquid fuel-synthesizing portion; and the acidic by-product is neutralized and removed with the temperature-controlling gas after having been flowed through the liquid fuel-synthesizing portion.

### B-1. Step I

In the step I, the raw material gas containing at least hydrogen and the carbon oxide is supplied to the liquid fuel-synthesizing portion including: the catalyst that advances the conversion reaction from the raw material gas to the liquid fuel; and the water vapor separation membrane that causes water vapor to permeate therethrough. As described in the section A, the liquid fuel-synthesizing portion **10** includes: the catalyst **12;** the water vapor separation membrane **14;** the non-permeation-side space **10A;** and the permeation-side space **10B.** The raw material gas is supplied from the first inflow port **s1** to the non-permeation-side space **10A** of the liquid fuel-synthesizing portion 10.

As described above, the raw material gas contains at least hydrogen and the carbon oxide. The concentrations of hydrogen and the carbon oxide in the raw material gas are as described above. The concentration of the acidic by-product in the raw material gas (when two or more kinds of acidic byproducts are present, their total concentration) is preferably as low as possible, and is, for example, 30 ppm or less, preferably 20 ppm or less.

### B-2. Step II

In the step II, the conversion reaction is advanced, and the remaining raw material gas, which contains hydrogen and the carbon oxide that are unreacted, and the acidic by-product, is recovered from the liquid fuel-synthesizing portion.

The conversion reaction advances in the catalyst **12.** Thus, the liquid fuel and the water vapor are produced. Specifically, as represented by the reaction formula (2), methanol and the water vapor are produced by subjecting a raw material gas containing CO₂ and hydrogen to catalytic hydrogenation in the presence of the catalyst. In addition, at this time, a trace amount of an acidic component (acidic by-product) is produced as a by-product except the water vapor. Reaction conditions are as described in the section A.

The liquid fuel produced in the non-permeation-side space **10A** flows out to the non-permeation-side gas-recovering pipe **31** through the first outflow port **d1** together with the remaining raw material gas containing hydrogen and the carbon oxide that are unreacted, and the acidic by-product, and the fuel is subjected to gas-liquid separation from the remaining raw material gas by the first condenser **32** and the first drain trap **33.** Thus, the liquid fuel is recovered from the liquid fuel-recovering pipe **34,** and the remaining raw material gas is recovered from the raw material gas-circulating pipe **35.**

### B-3. Step III

In the step III, the acidic by-product is removed from the remaining raw material gas.

The liquid fuel production system **1A** is configured to prepare the raw material gas through the mixing of the sweeping gas and the remaining raw material gas that have each been separately recovered from the liquid fuel-synthesizing portion **10.** Accordingly, when the sweeping gas containing the amine compound is used, the acidic by-product incorporated into the remaining raw material gas can be removed by being neutralized with the amine compound. A procedure therefor is specifically as described below.

In the liquid fuel production system **1A,** the water vapor produced in the non-permeation-side space **10A** and part of hydrogen in the raw material gas permeate through the water vapor separation membrane **14** to flow into the permeation-side space **10B.** The sweeping gas containing the amine compound, and optionally, hydrogen and/or the carbon oxide flows into the permeation-side space **10B** through the second inflow port **s2.** The water vapor and hydrogen that have flowed into the permeation-side space **10B** are swept by the sweeping gas, and flow out as an exhaust gas to the neutralizing raw material gas-supplying pipe (exhaust gas-recovering pipe) **22** through the second outflow port **d2.** The raw material gas-circulating pipe **35** merges with the neutralizing raw material gas-supplying pipe **22** in the mixing portion **24,** and hence the exhaust gas and the remaining raw material gas are mixed. Thus, the acidic by-product derived from the remaining raw material gas is neutralized with the amine compound derived from the sweeping gas incorporated into the exhaust gas. The mixed gas of the exhaust gas and the remaining raw material gas passes through the second condenser **26a** and the second drain trap **26b** in the stated order. During the passage, the water vapor is condensed and subjected to gas-liquid separation, followed by its capture (removal) from the moisture-capturing pipe **26c.** It is preferred that the neutralized product produced by the neutralization be dissolved in the condensed water, and be removed from the moisture-capturing pipe **26c** together with the condensed water.

### B-4. Step IV

In the step IV, the remaining raw material gas after the removal of the acidic by-product is resupplied as part of the raw material gas to the liquid fuel-synthesizing portion. Specifically, the temperature and pressure of the mixing gas after the removal of its moisture in the step III, which serves as the raw material gas, are adjusted to desired values in the pressure-boosting portion **28,** and then, the gas is supplied to the liquid fuel-synthesizing portion **10.** As described above, the mixed gas after the removal of the moisture in the step III may be mixed with hydrogen and/or the carbon oxide as required so that the raw material gas may be prepared in desired composition.

### Industrial Applicability

The liquid fuel production system according to the embodiment of the present invention may be suitably used in the production of a liquid fuel such as methanol.

### Reference Signs List

- **1A-D**: liquid fuel production system
- **10**: liquid fuel-synthesizing portion
- **20**: raw material gas-supplying portion
- **30**: raw material gas-circulating portion
- **40**: sweeping gas-supplying portion

## Claims

1. A liquid fuel production system, comprising:
a liquid fuel-synthesizing portion configured to advance a conversion reaction from a raw material gas containing at least hydrogen and a carbon oxide to a liquid fuel;
a raw material gas-supplying portion configured to supply the raw material gas to the liquid fuel-synthesizing portion; and
a raw material gas-circulating portion configured to resupply a remaining raw material gas, which contains the hydrogen and the carbon oxide that are unreacted, and an acidic by-product of the conversion reaction, from the liquid fuel-synthesizing portion to the raw material gas-supplying portion,
wherein the raw material gas-supplying portion includes
a mixing portion configured to mix an amine compound and the remaining raw material gas in the presence of water vapor, and
a moisture-removing portion configured to remove a neutralized product of the amine compound and the acidic by-product together with condensed water of the water vapor.

2. The liquid fuel production system according to claim 1, wherein a neutralizing raw material gas containing the amine compound and the remaining raw material gas are mixed in the mixing portion.

3. The liquid fuel production system according to claim 1,
wherein the raw material gas further contains nitrogen, and
wherein the amine compound is ammonia produced from the hydrogen and the nitrogen in the liquid fuel-synthesizing portion.

4. The liquid fuel production system according to claim 2, further comprising a gas-capturing portion configured to capture carbon dioxide from air or a biogas,
wherein a gas containing the carbon dioxide supplied from the gas-capturing portion is used as a constituent component for the neutralizing raw material gas.

5. The liquid fuel production system according to claim 2,
wherein the liquid fuel-synthesizing portion includes a water vapor separation membrane configured to cause at least water vapor to permeate therethrough, and the liquid fuel-synthesizing portion separates a permeation-side gas, which is a gas that has permeated through the water vapor separation membrane from a non-permeation side thereof to a permeation side thereof, and which contains water vapor that is a by-product of the conversion reaction, and a non-permeation-side gas, which is a gas that has been free from permeating through the water vapor separation membrane, and which contains the liquid fuel, the hydrogen and the carbon oxide that are unreacted, and the acidic by-product, from each other,
wherein the liquid fuel production system further comprises a sweeping gas-supplying portion configured to supply a sweeping gas, which is a gas that sweeps the permeation-side gas, and which contains an amine compound, to the permeation side of the liquid fuel-synthesizing portion, and
wherein the permeation-side gas and the sweeping gas that have flowed out of the liquid fuel-synthesizing portion are supplied to the mixing portion.

6. The liquid fuel production system according to claim 5, wherein the sweeping gas further contains a carbon oxide and/or hydrogen.

7. The liquid fuel production system according to claim 5, wherein a concentration of the amine compound in the sweeping gas is 10 ppm or more.

8. The liquid fuel production system according to claim 3,
wherein the liquid fuel-synthesizing portion includes a water vapor separation membrane configured to cause at least water vapor and ammonia to permeate therethrough, and the liquid fuel-synthesizing portion separates a permeation-side gas, which is a gas that has permeated through the water vapor separation membrane from a non-permeation side thereof to a permeation side thereof, and which contains water vapor that is a by-product of the conversion reaction and the ammonia, and a non-permeation-side gas, which is a gas that has been free from permeating through the water vapor separation membrane, and which contains the liquid fuel, the hydrogen and the carbon oxide that are unreacted, and the acidic by-product, from each other,
wherein the liquid fuel production system further comprises a sweeping gas-supplying portion configured to supply a sweeping gas, which sweeps the permeation-side gas, to the permeation side of the liquid fuel-synthesizing portion, and
wherein the permeation-side gas and the sweeping gas that have flowed out of the liquid fuel-synthesizing portion are supplied to the mixing portion.

9. The liquid fuel production system according to claim 8, wherein a concentration of the ammonia in a mixed gas of the permeation-side gas and the sweeping gas is 10 ppm or more.

10. The liquid fuel production system according to claim 2,
wherein the liquid fuel-synthesizing portion includes a liquid fuel separation membrane configured to cause at least a liquid fuel to permeate therethrough, and the liquid fuel-synthesizing portion separates a permeation-side gas, which is a gas that has permeated through the liquid fuel separation membrane from a non-permeation side thereof to a permeation side thereof, and which contains the liquid fuel, and a non-permeation-side gas, which is a gas that has been free from permeating through the liquid fuel separation membrane, and which contains water vapor that is a by-product of the conversion reaction, the hydrogen and the carbon oxide that are unreacted, and the acidic by-product, from each other,
wherein the liquid fuel production system further comprises a sweeping gas-supplying portion configured to supply a sweeping gas, which is a gas that sweeps the permeation-side gas, and which contains an amine compound, to the permeation side of the liquid fuel-synthesizing portion, and
wherein the sweeping gas that has flowed out of the liquid fuel-synthesizing portion is supplied to the mixing portion.

11. The liquid fuel production system according to claim 10, wherein the sweeping gas further contains a carbon oxide and/or hydrogen.

12. The liquid fuel production system according to claim 10, wherein a concentration of the amine compound in the sweeping gas is 10 ppm or more.

13. The liquid fuel production system according to claim 3,
wherein the liquid fuel-synthesizing portion includes a liquid fuel separation membrane configured to cause at least a liquid fuel and ammonia to permeate therethrough, and the liquid fuel-synthesizing portion separates a permeation-side gas, which is a gas that has permeated through the liquid fuel separation membrane from a non-permeation side thereof to a permeation side thereof, and which contains the liquid fuel and the ammonia, and a non-permeation-side gas, which is a gas that has been free from permeating through the liquid fuel separation membrane, and which contains water vapor that is a by-product of the conversion reaction, the hydrogen and the carbon oxide that are unreacted, and the acidic by-product, from each other,
wherein the liquid fuel production system further comprises a sweeping gas-supplying portion configured to supply a sweeping gas, which sweeps the permeation-side gas, to the permeation side of the liquid fuel-synthesizing portion, and
wherein the permeation-side gas and the sweeping gas that have flowed out of the liquid fuel-synthesizing portion are supplied to the mixing portion.

14. The liquid fuel production system according to claim 13, wherein a concentration of the ammonia in a mixed gas of the permeation-side gas and the sweeping gas is 10 ppm or more.

15. The liquid fuel production system according to claim 2,
wherein the liquid fuel-synthesizing portion includes a first gas flow path having arranged therein a catalyst that advances the conversion reaction, and a second gas flow path through which a temperature-controlling gas that controls a temperature of the first gas flow path flows,
wherein the liquid fuel production system further comprises a temperature-controlling gas-supplying portion configured to supply a temperature-controlling gas containing an amine compound to the second gas flow path of the liquid fuel-synthesizing portion, and
wherein the temperature-controlling gas that has flowed out of the liquid fuel-synthesizing portion is supplied to the mixing portion.

16. The liquid fuel production system according to claim 15, wherein the temperature-controlling gas further contains a carbon oxide and/or hydrogen.

17. The liquid fuel production system according to claim 15, wherein a concentration of the amine compound in the temperature-controlling gas is 10 ppm or more.

18. A method of producing a liquid fuel, comprising:
supplying a raw material gas containing at least hydrogen and a carbon oxide to a liquid fuel-synthesizing portion including a catalyst that advances a conversion reaction from the raw material gas to a liquid fuel;
advancing the conversion reaction, and recovering a remaining raw material gas, which contains the hydrogen and the carbon oxide that are unreacted, and an acidic by-product, from the liquid fuel-synthesizing portion;
removing the acidic by-product from the remaining raw material gas; and
resupplying the remaining raw material gas after the removal of the acidic by-product as part of the raw material gas to the liquid fuel-synthesizing portion,
wherein the removing the acidic by-product from the remaining raw material gas includes mixing an amine compound and the remaining raw material gas in the presence of water vapor to neutralize the acidic by-product with the amine compound.

19. The production method according to claim 18, wherein the removing the acidic by-product from the remaining raw material gas includes mixing a neutralizing raw material gas containing the amine compound after having been supplied to the liquid fuel-synthesizing portion and having flowed through the liquid fuel-synthesizing portion, and the remaining raw material gas.

20. The production method according to claim 18,
wherein the raw material gas further contains nitrogen, and
wherein the amine compound is ammonia produced from the hydrogen and the nitrogen in the liquid fuel-synthesizing portion.

21. The production method according to claim 19, wherein the neutralizing raw material gas is prepared by using a gas containing carbon dioxide captured from air or a biogas.

22. The production method according to claim 19,
wherein the liquid fuel-synthesizing portion includes a water vapor separation membrane configured to cause at least water vapor to permeate therethrough,
wherein water vapor that is a by-product of the conversion reaction is caused to permeate from a non-permeation side of the water vapor separation membrane to a permeation side thereof, is swept by a sweeping gas containing the amine compound, and is recovered as an exhaust gas from the liquid fuel-synthesizing portion, and
wherein the exhaust gas and the remaining raw material gas are mixed to neutralize the acidic by-product in the remaining raw material gas with the amine compound.

23. The production method according to claim 19,
wherein the liquid fuel-synthesizing portion includes a liquid fuel separation membrane configured to cause at least a liquid fuel to permeate therethrough,
wherein the liquid fuel is caused to permeate from a non-permeation side of the liquid fuel separation membrane to a permeation side thereof, is swept by a sweeping gas containing the amine compound, and is recovered as a product gas from the liquid fuel-synthesizing portion, and water vapor that is a by-product of the conversion reaction is recovered,
wherein the product gas is separated into the liquid fuel and the sweeping gas, and
wherein the sweeping gas, the remaining raw material gas, and the water vapor are mixed to neutralize the acidic by-product in the remaining raw material gas with the amine compound.

24. The production method according to claim 19,
wherein a temperature-controlling gas containing the amine compound for controlling a reaction temperature of the conversion reaction is flowed through the liquid fuel-synthesizing portion, and
wherein the temperature-controlling gas after having flowed through the liquid fuel-synthesizing portion and the remaining raw material gas are mixed to neutralize the acidic by-product in the remaining raw material gas with the amine compound.

25. The production method according to claim 20,
wherein the liquid fuel-synthesizing portion includes a water vapor separation membrane configured to cause at least water vapor and ammonia to permeate therethrough,
wherein water vapor that is a by-product of the conversion reaction and the ammonia are caused to permeate from a non-permeation side of the water vapor separation membrane to a permeation side thereof, are swept by a sweeping gas, and are recovered as an exhaust gas from the liquid fuel-synthesizing portion, and
wherein the exhaust gas and the remaining raw material gas are mixed to neutralize the acidic by-product in the remaining raw material gas with the ammonia.

26. The production method according to claim 20,
wherein the liquid fuel-synthesizing portion includes a liquid fuel separation membrane configured to cause at least a liquid fuel and ammonia to permeate therethrough,
wherein the liquid fuel and the ammonia are caused to permeate from a non-permeation side of the liquid fuel separation membrane to a permeation side thereof, are swept by a sweeping gas, and are recovered as a product gas from the liquid fuel-synthesizing portion, and water vapor that is a by-product of the conversion reaction is recovered together with the remaining raw material gas, and
wherein the liquid fuel is separated from the product gas, and then, the remaining is mixed with the remaining raw material gas and the water vapor to neutralize the acidic by-product in the remaining raw material gas with the ammonia.
